# EUROPEAN PATENT APPLICATION

(11) **EP 4 455 671 A1**
(43) Date of publication of application: **30.10.2024**
(21) Application number: 23170167.3
(22) Date of filing: 26.04.2023
(51) Int. Cl.: G01N 33/68

(54) **SINGLE TUBE THERMAL PROTEOME PROFILING WITH UNIFORM PROGRESSION**

(71) Applicant: Stichting Radboud Universiteit, 6525 XZ Nijmegen (NL)
(72) Inventor: VERMEULEN, Michiel, 6525XZ Nijmegen (NL); ZIJLMANS, Dick, 6525XZ Nijmegen (NL)
(74) Representative: V.O.

(57) **Abstract**

The invention relates to methods of identifying at least one protein that exhibits altered thermal stability upon addition of a compound.

## Description

FIELD The invention relates to methods of identifying one or more proteins that exhibit altered thermal stability upon addition of a compound using a thermal shift assay.

### BACKGROUND

Advances in the field of drug development have provided a wide range of therapies for a multitude of diseases. However, a major issue that remains is the off-target effects some therapies have in certain patients, resulting in unfavorable disease outcomes. Thermal proteome profiling (TPP) was originally developed as a method to identify the direct and indirect targets of newly developed drug molecules (Franken et al., 2015. Nature Protocols 10: 1567-1593), potentially helping to reduce failure of compounds during different stages of drug discovery. TPP measures changes in thermal stability between any two conditions and uses that to infer a gain or loss of interactions, i.e. proteins that bind to a particular compound will show an increased stability and will be more resistant to thermal denaturation. More recently, TPP has been applied more broadly as a tool to examine changes in protein interactions between any two conditions, including drug binding, protein-protein interactions and nucleic acid-protein interactions (Mateus et al., 2020. Mol Syst Biol 16: e9232).

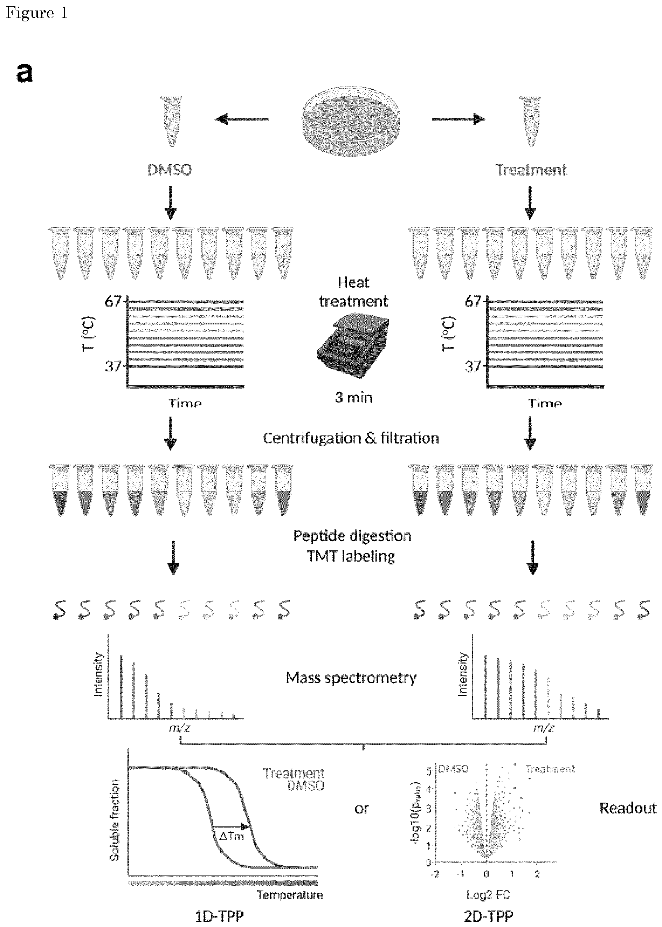

In a conventional TPP workflow, cells are treated with the compound of interest and harvested (see Figure 1A). The cells are split into multiple samples, which are each exposed to a distinct set temperature for a set amount of time. The cells are subsequently lysed and the lysate is centrifuged and filtered to remove precipitated proteins. Equal amounts of filtered lysate for each temperature are then digested, labeled with Tandem Mass Tag (TMT), pooled by either replicate-condition (1D-TPP) or temperature (2D-TPP), and measured by mass spectrometry to generate melting curves for each protein in the proteome. Shifts in melting curves between conditions function as read-out for changes in protein interactions and allow for identification of proteins that are affected by the compound of interest.

One major drawback of TPP, which prevents it from being used as a tool for high-throughput drug screenings, is the high instrument time and input material required, due to the measuring of protein abundances at multiple temperatures (see Figure 1A).

There is thus a need to provide methods and means that allow screening of multiple samples in reduced time. Furthermore, said methods and means preferably allow TPP starting with reduced amounts of input material.

### BRIEF DESCRIPTION OF THE INVENTION

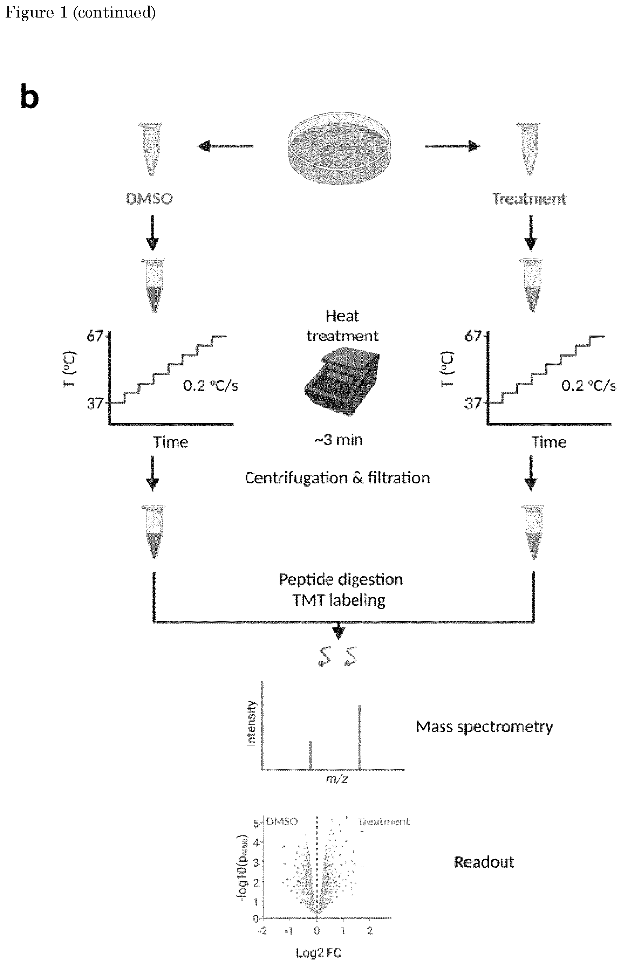

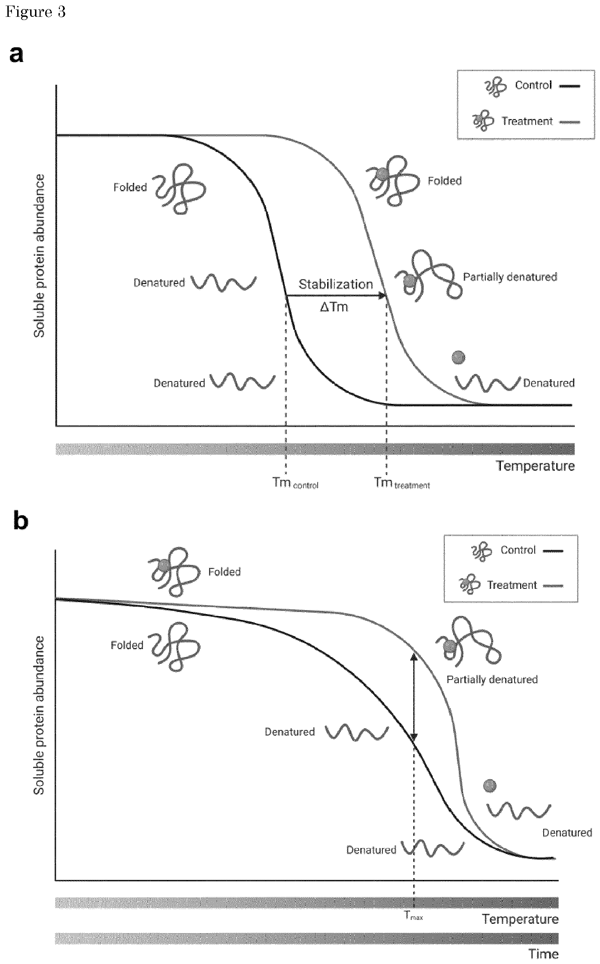

By changing the heat treatment program such that the temperature exposure and accordingly protein denaturation is incremental as opposed to constant, changes in protein stability between conditions can be assessed in a single sample (Figure 1B). This methodology, termed Single-tube TPP with Uniform Progression (STPP-UP) reduces the instrument time required 10-fold and the input material needed between 5- and 10-fold, compared to conventional TPP. In STPP-UP, a single sample of cells is exposed to an incrementally increasing temperature until a Tmax is reached, as opposed to using multiple samples, each exposed to a distinct temperature. In this fashion, if the denaturation rate between two conditions is different at any temperature, this will result in a difference in protein abundance that will persist and potentially compound until Tmax is reached (Figure 3B). Optionally, a 37 °C or non-heated carrier sample can be included to adjust for base differences in expression between conditions.

The invention therefore provides a method of identifying at least one protein that exhibits altered thermal stability upon addition of a compound, comprising the steps of a) incubating at least one container comprising a plurality of proteins with the compound, and at least one container comprising the plurality of proteins with a control; b) exposing the containers to an increasing temperature; c) digesting the proteins; d) labelling the digested proteins; and e) comparing the labelled digested proteins from the at least one container that was incubated with the compound with the labelled digested proteins from the at least one container that was incubated with the control, to identify at least one protein that exhibits altered thermal stability upon addition of the compound. Said altered thermal stability may indicate that the protein interacts with the compound.

In methods of the invention, step b) may be followed by a step of separating soluble from insoluble proteins, followed by digesting the soluble and/or the insoluble proteins in step c).

In methods of the invention, an aliquot, termed carrier, may be removed from the at least one container that was incubated with the compound after step a), which aliquot is used to adjust for differences between replicates. A second, equal, aliquot of the at least one container that was incubated with the compound after step a) being termed tester. Said carrier and tester aliquots may be positioned in separate containers for further incubation as is detailed herein below.

In methods of the invention, the carrier aliquot may be incubated at a temperature between 20 and 50 °C for between 10 seconds and 10 minutes, such as between 30 seconds and 5 minutes, including 1 minute, 2 minutes, 3 minutes, and 4 minutes. Preferably, the carrier aliquot is kept at room temperature until the tester aliquots have been heat treated, after which time both aliquots are put on ice.

A method of the invention may comprise identifying at least one protein that exhibits altered thermal stability upon addition of a compound for a plurality of conditions whereby, for each condition, at least one container comprising a plurality of proteins is incubated with the compound, and at least one container comprising the plurality of proteins is incubated with a control. In such methods, for each condition, an aliquot may be removed from the at least one container that was incubated with the compound after step a), which aliquot is used as carrier to adjust for differences between conditions and, if appropriate, between replicates.

In methods of the invention, the plurality of proteins may be present in one or more cells such as mammalian cells, including human cells.

In methods of the invention, the at least one protein may interact with the compound.

In methods of the invention, the control is or comprises the solvent in which the compound is solubilized, such as dimethyl sulfoxide.

In methods of the invention, the digested proteins are labelled with a tandem mass tag (TMT) label.

In methods of the invention, the labelled samples may be combined before comparing the labelled digested proteins from the at least one container that was incubated with the compound with the labelled digested proteins from the at least one container that was incubated with the control, to identify at least one protein that exhibits altered thermal stability upon addition of the compound.

In methods of the invention, the comparison of the labelled digested proteins from the at least one container that was incubated with the compound with the labelled digested proteins from the at least one container that was incubated with the control, may be performed by mass spectrometry, such as reverse-phase liquid chromatography (RP-LC) coupled to tandem mass spectrometry (MS/MS).

In methods of the invention, the tester aliquots, or the containers comprising the tester aliquots, may be exposed to an increasing temperature between 30 °C and 75 °C, such as between 35 °C and 60 °C, between 36 °C and 58 °C, between 37 °C and 57 °C, between 38 °C and 56 °C, between 39 °C and 55 °C, or between 40 °C and 54 °C.

In methods of the invention, tester aliquots, or the containers comprising the tester aliquots, may be exposed to a steadily increasing temperature between 30 °C and 75 °C, at a rate between 0.05 °C and 5 °C per second.

In methods of the invention, the proteins may be digested with a combination of trypsin and LysC endoproteinase.

### FIGURE LEGENDS

Figure 1. Comparing workflows of TPP and STPP-UP. a. Example of a TPP workflow. Cells of two different conditions are harvested and distributed over multiple tubes. Each tube is exposed to a distinct set temperature for 3 minutes, followed by 3 minutes of incubation at room temperature (RT) and lysis. Lysates are then centrifuged and filtered to remove precipitated proteins and equal volumes of filtered lysates are digested and labeled with TMT. TMT labeling can be done per replicate-condition (1D-TPP) or temperature (2D-TPP). Using 1D-TPP allows for more accurate determination of melting curves, whereas 2D-TPP allows for more accurate comparison between replicates/conditions for each temperature. After labeling, samples are pooled and subjected to mass spectrometry. As readout, 1D-TPP uses shifts in Tm obtained from melting curves to assess changes in stability whereas 2D-TPP uses stability scores to determine changes in stability. Stability scores are calculated as the sum of the log-transformed fold changes between conditions at each temperature adjusted to the relative fold changes measured at the first two temperatures. b. Example of a STPP-UP workflow. Cells of two different conditions are harvested and added to a single tube or optionally a 37oC or non-heated carrier sample. The sample is then exposed to an incrementally increasing temperature until a Tmax is reached, followed by 3 minutes of incubation at room temperature (RT) and lysis. Lysates are then centrifuged and filtered to remove precipitated proteins and equal volumes of filtered lysates are digested and labeled with TMT. In STPP-UP, all replicates for both test and carrier samples for both conditions can be labeled together and combined into a single sample using TMTpro 16-plex. Samples are then subjected to mass spectrometry. As a readout, STPP-UP uses changes in abundance to determine changes in stability.

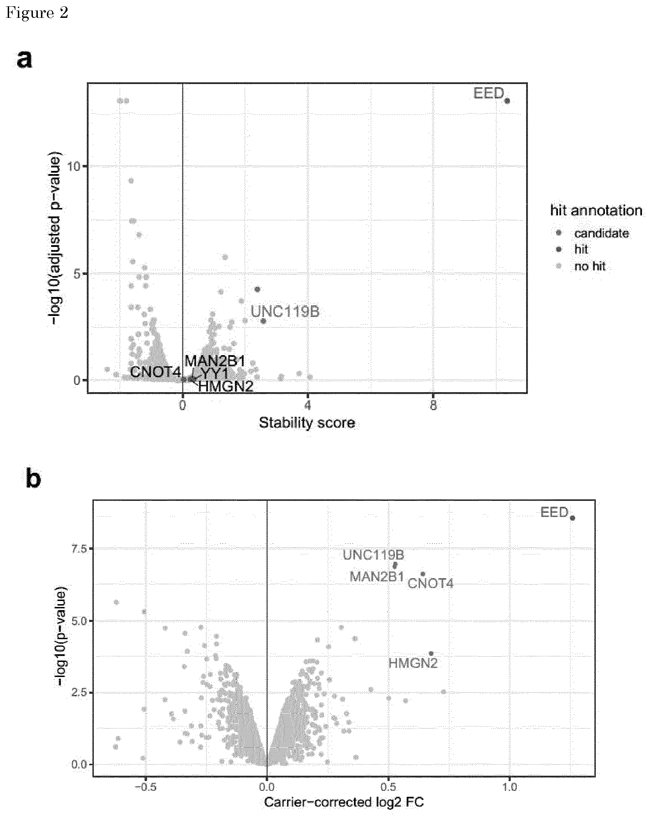

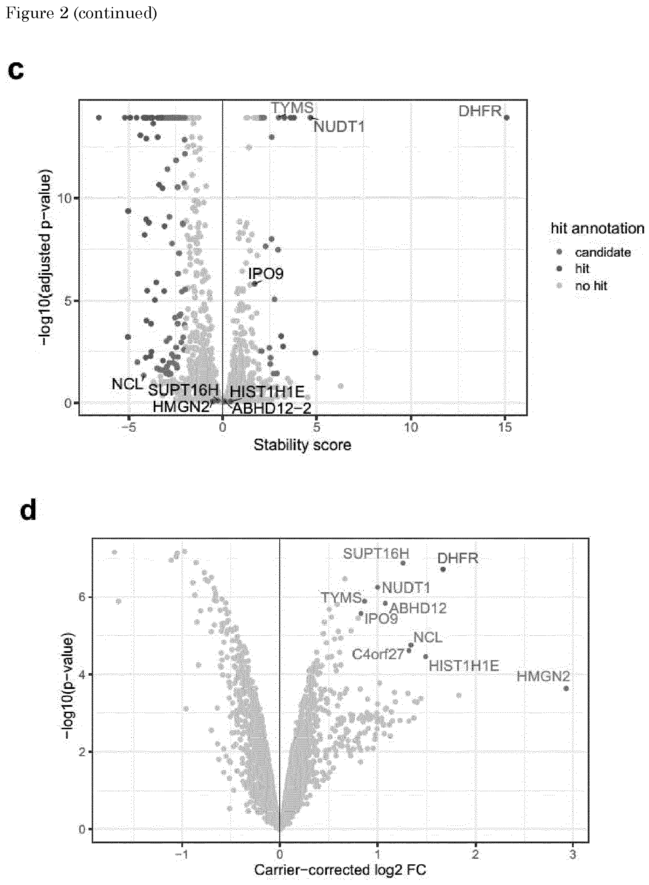

Figure 2. STPP-UP identifies direct drug targets similar to TPP. a. Volcano plot of stability scores calculated from the TPP experiment in E 14 mouse embryonic stem cells (mESCs). Cells were treated with 10 µM of an allosteric polycomb repressive complex 2 (PRC2) inhibitor, EED226, or DMSO for 1 hour. Two biological replicates were used per condition. Proteins with a stability score >3 and a false discovery rate (FDR) <0.01 are indicated and were designated as "hit". Proteins with a stability score >2 and an FDR <0.05 were designated as "candidate". b. Volcano plot of STPP-UP showing changes in protein abundance between EED226- and DMSO-treated E14 mESCs after carrier correction. EED226 treatment was done at 10 µM for 1 hour. Tmax was set at 57 °C. Three biological replicates were used per condition. EED (direct target) and other significantly enriched proteins (log2 FC > 0.5, p-value <0.001, students t-test) are indicated, c. Volcano plot of stability scores calculated from the TPP experiment in LS 174T cells. Cells were treated with 10 µM C1 or DMSO for 1 hour. Three biological replicates were used per condition. Proteins with a stability score >3 and an FDR <0.01 were designated as "hit", including NUTD1 and DHFR. Proteins with a stability score >2 and an FDR <0.05 were designated as "candidate", including TYMS. d. Volcano plot of STPP-UP showing changes in protein abundance between C1- and DMSO-treated LS 174T cells after carrier correction. C1 treatment was done at 10 µM for 1 hour. Tmax was set at 57 °C. Three biological replicates were used per condition. Dihydrofolate reductase (DHFR; direct target) is indicated, and several other significantly enriched proteins (log2 FC > 0.5, p-value <0.001, students t-test) are provided.
Figure 3. a. Principle behind TPP. Proteins that bind to a compound of interest are more resistant to thermal denaturation. By profiling protein denaturation at different temperatures, we can generate a melting curve that we can compare between two conditions. Shifts in the melting curves and therefore changes in stability are used as a readout for gain or loss of interactions, b. Principle behind STPP-UP. Samples are exposed to a incrementally increasing temperature, which will result in an incrementally increasing denaturation rate. Differences in denaturation rate at any temperature in the increment will compound when Tmax is reached.

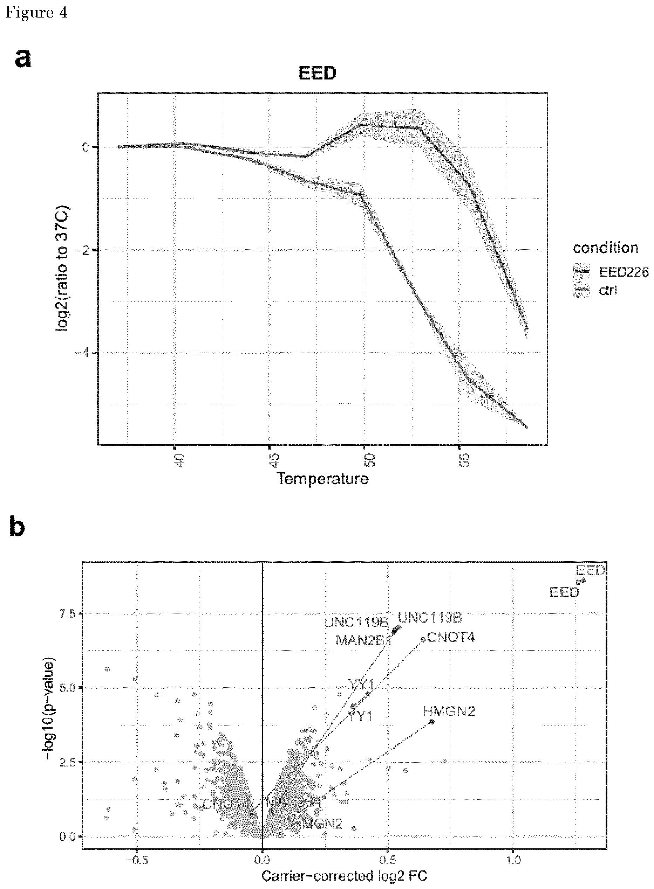

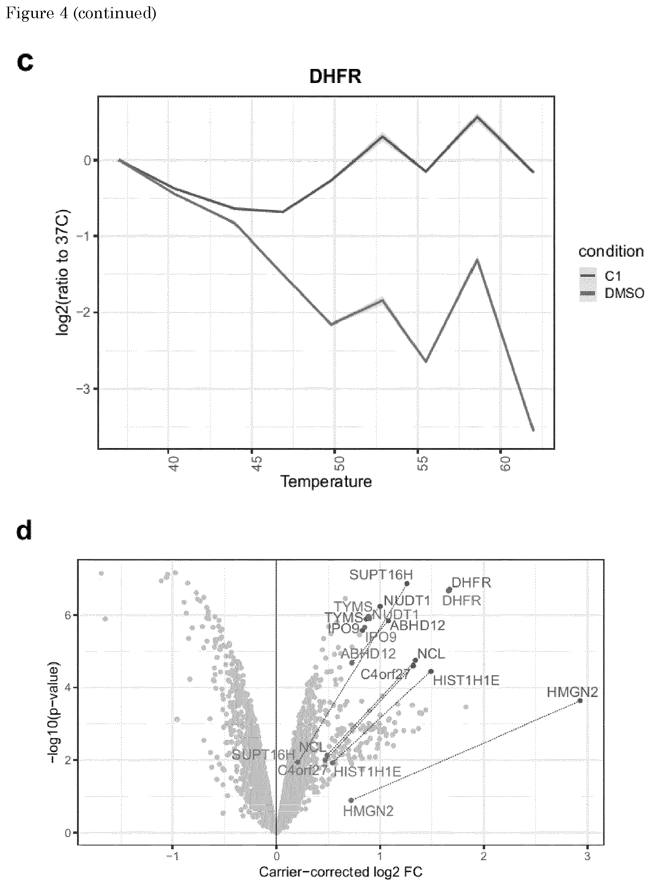

Figure 4. a. TPP melting curves for FED in E14 mESCs after 10 µM EED226 or DMSO treatment for 1 hour. Data are normalized to the 37 °C sample for each condition. Shaded regions shows the standard error. Two biological replicates were used per condition. b. Volcano plot from Figure 2B showing the effect of carrier-correction on significantly enriched proteins. For each protein, their carrier-corrected values are show in grey, uncorrected values are shown in black. Lines indicate shifts between corrected and uncorrected. c. TPP melting curves for DHFR in LS 174T cells after 10 µM C1 or DMSO treatment for 1 hour. Data are normalized to the 37 °C sample for each condition. Shaded regions shows the standard error. Three biological replicates were used per condition. d. Volcano plot from Figure 2D showing the effect of carrier-correction on significantly enriched proteins. For each protein, their carrier-corrected values are show in black, uncorrected values are shown in grey. Dotted lines indicate shifts between corrected and uncorrected.

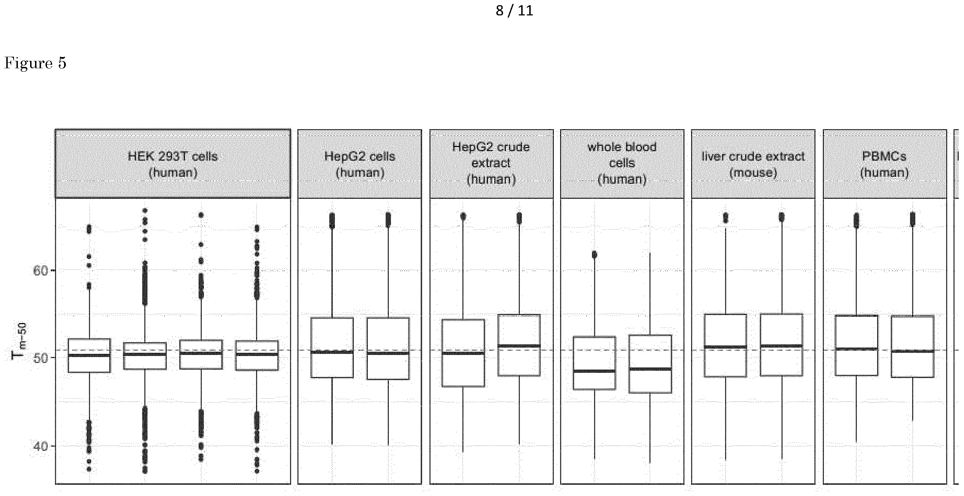

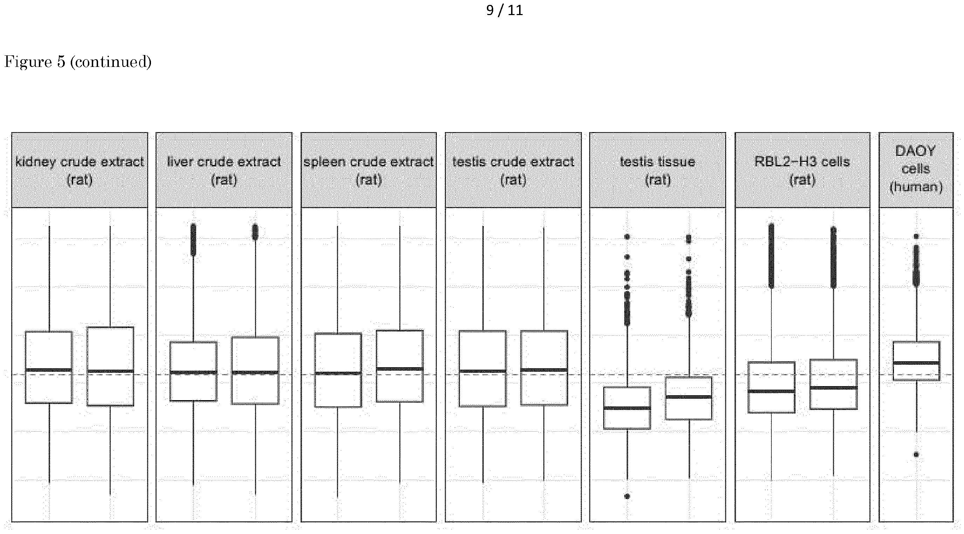

Figure 5. Boxplots showing the average Tm-50 in mouse and human cells. Data on mouse was taken from EED226-treated E14 mESCs TPP experiment. Data on human was taken from van der Krift et al., 2023 (BioRxiv 2023; 10.1101/2023.02.26.530079). For accuracy, only proteins that had a complete melting curve (measured protein abundance at all temperatures) were included. was used for as determined in previously published TPP datasets in mouse and human. The boxplots show the interquartile range (box limits showing the 25th and 75th percentile) and median (centre line). Whiskers indicate 1.5x the interquartile range.

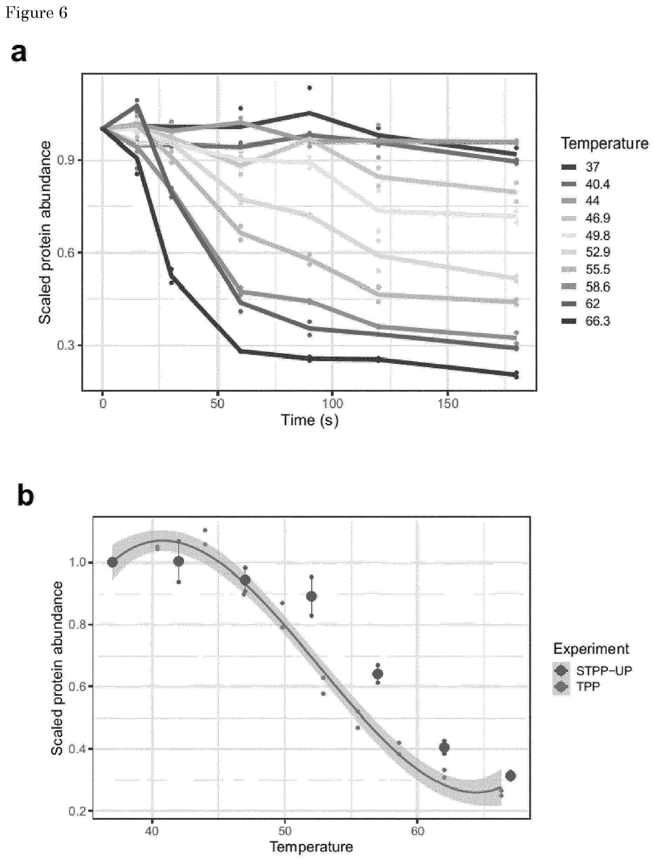

Figure 6. a. Time course showing protein denaturation in E 14 mESCs at the different temperatures used in the TPP range. Protein abundances were determined by BCA. For each temperature, samples were scaled to their respective untreated (t=0) samples. Two biological replicates were used per condition. b. Protein abundances as determined by BCA for STPP-UP at different Tmax compared to conventional TPP (37 °C-67 °C). Two biological replicates were used per condition. For STPP-UP, samples were scaled to 37 °C untreated samples; For TPP, samples were normalized to the 37 °C samples.

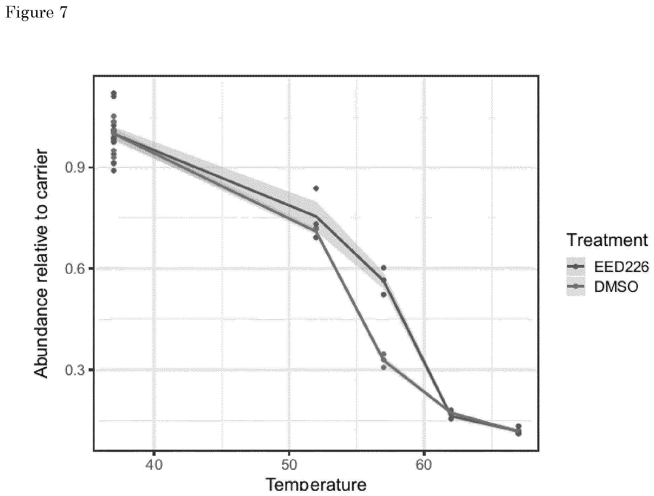

Figure 7. Line plot showing relative abundance of FED after STPP-UP in R1 mESCs using different Tmax. Cells were treated with 10 µM EED226 or DMSO for 1 hour. For each Tmax, the intensity of the test sample was scaled to the mean intensity of their respective carrier. Shaded regions shows the standard error. Three biological replicates were used for each condition.

### DETAILED DESCRIPTION OF THE INVENTION

The invention is described by the following aspects. For purposes of clarity and a concise description, herein features have been described as part of the same or different embodiments, but it will be clear that the scope of protection of the invention can encompass embodiments with combinations of any or all of the features described. It will be appreciated that the embodiments shown have the same or similar components, apart from where they have been described as different.

The word 'comprising' does not preclude the presence of other features or steps than those specified in a claim. Further, the words 'a(n)' and 'one' should not be construed as limiting to 'just one', but instead are used to indicate 'at least one', and do not preclude plurality.
1. A method of identifying at least one protein that exhibits altered thermal stability upon addition of a compound, comprising the steps of:
   a) incubating at least one container comprising a plurality of proteins with the compound, and at least one container comprising the plurality of proteins with a control;
   b) exposing the containers to an increasing temperature;
   c) digesting the proteins;
   d) labelling the digested proteins; and
   e) comparing the labelled digested proteins from the at least one container that was incubated with the compound with the labelled digested proteins from the at least one container that was incubated with the control, to identify at least one protein that exhibits altered thermal stability upon addition of the compound.
2. The method of aspect 1, wherein step b) is followed by a step of separating soluble from insoluble proteins, followed by digesting the soluble and/or the insoluble proteins in step c).
3. The method of aspect 1 or aspect 2, wherein the at least one container comprising a plurality of proteins that are incubated with the compound refers to a total of 2 - 20 replicate containers.
4. The method of any one of aspects 1-3, wherein the at least one container comprising a plurality of proteins that are incubated with the control refers to a total of 2 - 20 replicate containers.
5. The method of any one of aspects 1-4, wherein the number of replicate containers is independently is chosen from 2, 3, 4, 5, 6, 7, 8, 9, 10, 11, 12, 13, 14, 15, 16, 17, 18, 19 or 20 replicate containers.
6. The method of any one of aspects 1-5, wherein an aliquot, termed carrier, is removed from the at least one container that was incubated with the compound after step a), which aliquot is used to adjust for differences between replicates and/or conditions, while a second, equal aliquot from the container that was incubated with the compound after step a) is termed tester.
7. The method of aspect 6, wherein the carrier aliquot is incubated at a temperature for at least 10 seconds.
8. The method of aspect 6 or 7, wherein the carrier aliquot is incubated at a temperature for between 10 seconds and 10 minutes, such as between 30 seconds and 5 minutes, including 1 minute, 2 minutes, 3 minutes, and 4 minutes, preferably for the same period of time that the tester aliquots are being heat treated.
9. The method of any one of aspects 6-8, wherein the temperature is a stable temperature.
10. The method of any one of aspects 6-9, wherein the carrier aliquot is incubated at a stable temperature between 20 and 50 °C.
11. The method of any one of aspects 6-10, wherein the carrier aliquot is incubated at a stable temperature between 21 °C and 45 °C, such as between 21 °C and 30 °C, between 22 °C and 28 °C, between 23 °C and 26 °C, between 24 °C and 25 °C, or about 25 °C, preferably at room temperature.
12. The method of any one of aspects 1-11, comprising identifying at least one protein that exhibits altered thermal stability upon addition of a compound for a plurality of conditions whereby, for each condition, at least one container comprising a plurality of proteins is incubated with the compound, and at least one container comprising the plurality of proteins is incubated with a control.
13. The method of aspect 12, whereby, for each condition, an aliquot is removed from the at least one container that was incubated with the compound after step a), which aliquot is used to adjust for differences between conditions and, if appropriate, between replicates.
14. The method of any one of aspects 1-13, wherein the plurality of proteins is present in one or more units.
15. The method of aspect 14, wherein each of the units comprises a membrane.
16. The method of any one of aspects 1-15, wherein the plurality of proteins is present in one or more cells.
17. The method of aspect 16, wherein the one or more cells are mammalian cells, such as human cells.
18. The method of any one of aspects 1-17, wherein the at least one protein interacts with the compound.
19. The method of any one of aspects 1-18, wherein the control is or comprises the solvent in which the compound is solubilized.
20. The method of any one of aspects 1-19, wherein the control is or comprises dimethyl sulfoxide.
21. The method of any one of aspects 1 to 20, wherein the digested proteins are labelled with a tandem mass tag (TMT) label.
22. The method of any one of aspects 1 to 21, wherein a different TMT label is used for each container.
23. The method of any one of aspects 1 to 22, wherein a different TMT label is used for each replicate.
24. The method of any one of aspects 1 to 23, wherein a different TMT label is used for each condition.
25. The method of any one of aspects 1 to 24, wherein a different TMT label is used for tester and carrier.
26. The method of any one of aspects 1 to 25, wherein all labelled samples are combined before comparing the labelled digested proteins from the at least one container that was incubated with the compound with the labelled digested proteins from the at least one container that was incubated with the control, to identify at least one protein that exhibits altered thermal stability upon addition of the compound.
27. The method of any one of aspects 1 to 26, wherein the comparison of the labelled digested proteins from the at least one container that was incubated with the compound with the labelled digested proteins from the at least one container that was incubated with the control, is performed by mass spectrometry, such as reverse-phase liquid chromatography (RP-LC) coupled to tandem mass spectrometry (MS/MS).
28. The method of any one of aspects 1 to 27, wherein the tester aliquots, or the containers comprising the tester aliquots, are exposed to an increasing temperature between 30 °C and 75 °C.
29. The method of any one of aspects 1 to 28, wherein the tester aliquots, or the containers comprising the tester aliquots, are exposed to an increasing temperature between 35 °C and 59 °C, such as between 36 °C and 58 °C, between 37 °C and 57 °C, between 38 °C and 56 °C, between 39 °C and 55 °C, or between 40 °C and 54 °C.
30. The method of any one of aspects 1 to 29, wherein the tester aliquots, or the containers comprising the tester aliquots, are exposed to a steadily increasing temperature between 30 °C and 75 °C.
31. The method of any one of aspects 1 to 30, wherein the tester aliquots, or the containers comprising the tester aliquots, are exposed to a steadily increasing temperature between 30 °C and 75 °C, at a rate between 0.05 °C and 5 °C per second.
32. The method of any one of aspects 1 to 31, wherein the tester aliquots, or the containers comprising the tester aliquots, are exposed to a steadily increasing temperature between 30 °C and 60 °C, at a rate between 0.1 °C and 3 °C per second, such as between 0.2 °C and 2 °C per second, or between 0.3 °C and 1 °C per second.
33. The method of any one of aspects 1 to 32, wherein the compound is an enzymatic inhibitor or an enzymatic activator.
34. The method of any one of aspects 1 to 33, wherein the compound is a cell-penetrating compound.
35. The method of any one of aspects 1 to 34, wherein the proteins are digested with an endoproteinase such as Arg-C (clostripain), Asp-N, chymotrypsin, Glu-C, trypsin and lysC.
36. The method of any one of aspects 1 to 35, wherein the proteins are digested with a recombinant endoproteinase.
37. The method of any one of aspects 1 to 36, wherein the proteins are digested with trypsin.
38. The method of any one of aspects 1 to 37, wherein the proteins are digested with a combination of trypsin and LysC endoproteinase.
39. The method of any one of aspects 1 to 38, wherein the at least one protein that exhibits altered thermal stability upon addition of the compound is identified by matching peptide fragment ion spectra to theoretical spectra generated from protein databases.
40. The method of any one of aspects 1 to 39, wherein the measuring time is 4-8 hours, accounting for fractionation of the sample.
41. The method of any one of aspects 1 to 40, wherein the input material for a single replicate is about 2 million cells.

### EXAMPLE

### Cell culture

E14 and R1 mESCs were cultured on 0.15% (w/v) gelatin-coated dishes in Dulbecco's Modified Eagle Medium (DMEM; Gibco) supplemented with 2 mM GlutaMAX (Gibco), 1 mM sodium pyruvate (Gibco), lx non-essential amino acids (Gibco), 50 U/mL penicillin-streptomycin (Gibco), 15% fetal bovine serum (FBS; HyClone), leukemia inhibitory factor (LIF; produced in-house) and 100 µM β-mercaptoethanol (Sigma-Aldrich). LS 174T colorectal cancer cells were cultured in RPMI 1640 medium (Gibco) supplemented with 50 U/mL penicillin-streptomycin and 10% FBS.

### Thermal proteome profiling

Thermal protein profiling was done as previously described (Becher et al., 2018. Cell 173: 1495-1507). In brief, cells were harvested after 1 hour of treatment with the compound of interest or DMSO, washed with PBS, and counted. Ten aliquots, each containing 10⁶ cells in 100 pL PBS, were distributed in a row of a 96-well PCR plate. After centrifugation (300 × g for 3 min) and removal of 80 µL of the supernatant, each aliquot was heated for three minutes to a distinct temperature (37°C, 40.4°C, 44°C, 46.9°C, 49.8°C, 52.9°C, 55.5°C, 68.6°C, 62°C, 66.3°C) in a PCR machine (Agilent SureCycler 8800), followed by incubation at room temperature (RT) for 3 min. Cells were lysed with 30 µL ice-cold lysis buffer (1.33% NP-40, 2.5 mM MgCl2, 1.6x protease inhibitors, 1.6x phosphatase inhibitors, 0.417 U/µL benzonase) on a shaker (500 rpm) at 4 °C for one hour. Samples were centrifuged at 300 × g for 3 min at 4°C to remove cell debris, and the supernatant was filtered at 300 × g for 3 min at 4°C through a 0.45-µm 96-well filter plate (Millipore, MSHVN4550) that was pre-wetted with plate-wash buffer (0.8% NP-40 in PBS) to remove protein aggregates. Of the flow-through, 25 µL was mixed with 2× sample buffer (180 mM Tris pH 6.8, 4% SDS, 20% glycerol, 0.1 g bromophenol blue) and kept at -20 °C until prepared for mass spectrometry analysis, while the remainder was used in a bicinchoninic acid assay (BCA; ThermoFisher Scientific), to determine the protein concentration. Samples were diluted to 0.5 µg/µL in 1x sample buffer based on the protein concentrations in the lowest two temperatures (37°C, 40.4°C).

### STPP- UP

Cells were harvested after 1 hour of treatment with the compound of interest or DMSO, washed with PBS, and counted. Two aliquots, each containing 10⁶ cells in 100 µL PBS, were distributed over a 96-well PCR plate. After centrifugation (300 × g for 3 min) and removal of 80 µL of the supernatant, one sample was heated to 37 °C for three minutes followed by incubation at RT for 3 min ("carrier"), while the other sample was incubated at 37°C for 5 seconds, heated to 57 °C at a rate of 0.2 °C/second and then incubated at RT for 3 minutes ("test"). Cells were transferred to ice and subsequently lysed with 30 µL ice-cold lysis buffer (1.33% NP-40, 2.5mM MgCl2, 1.6x protease inhibitors, 1.6x phosphatase inhibitors, 0.417 U/µL benzonase) and incubated on a shaker (500 rpm) at 4°C for one hour. Samples were centrifuged at 300 × g for 3 min at 4 °C to remove cell debris, and the supernatant was filtered at 300 × g for 3 min at 4 °C through a 0.45-µm 96-well filter plate (Millipore, MSHVN4550) that was pre-wetted with plate-wash buffer (0.8% NP-40 in PBS) to remove protein aggregates. Of the flow-through, 25µL was mixed with 2× sample buffer (180 mM Tris pH 6.8, 4% SDS, 20% glycerol, 0.1 g bromophenol blue) and kept at -20 °C until prepared for mass spectrometry analysis, while the remainder was used for a BCA (ThermoFisher Scientific) to determine protein concentrations in the carrier samples. Sample buffer-mixed carrier samples were diluted to 0.5 pg/pL with a 1:1 ratio of plate wash buffer:2x sample buffer. An equal volume of diluent was added to the test samples.

### MS sample preparation

Proteins were digested as previously described (Mateus et al., 2020. Mol Syst Biol 16: e9232). Briefly, for each condition/temperature, 20 µl sample (10 pg protein) was added to 40 µl bead suspension (5% 1:1 Sera-Mag Speed Beads pre-diluted 1:10 in H₂O (Thermo Fischer Scientific; 4515-2105-050250 & 6515-2105-050250), 3.5% formic acid, 70% EtOH) on a 0.45-µm 96-well filter plate (Millipore, MSHVN4550) and incubated on a shaker (500 rpm) for 15 min at RT. Beads were washed four times with 70% ethanol and proteins were digested overnight in 40 µl digest solution (5 mM chloroacetamide, 1.25 mM tris(2-carboxethyl)phosphine (TCEP), 200 ng trypsin, and 200 ng LysC in 100 mM HEPES pH 8). After digestion, samples were centrifuged to collect peptides. Peptides were vacuum-dried, reconstituted in 10 µl of water, and labeled for 1 hour at RT with either 80 pg of TMT10plex or 50 pg of TMTpro 16plex (ThermoFisher Scientific) dissolved in 4 µl or 2 µl of acetonitrile respectively. An overview of the labels used for each experiment can be found in Table 1. The reaction was quenched with 5 µl of 5% hydroxylamine and samples were combined.

### Sample cleanup, fractionation and measurement

For TPP, pooled samples were desalted using an OASIS plate (Waters; 186001828BA). After activation with 80% acetonitrile (ACN), wells were equilibrated with 0.05% formic acid (FA)/water. Samples were loaded and washed twice with 0.05% FA/water. Samples were eluted with 80% ACN and then prepared for high pH fractionation. Offline high pH reverse phase fractionation was carried out on an Agilent 1200 Infinity high-performance liquid chromatography system, equipped with a Gemini C18 column (3 pm, 110 Å, 100 × 1.0 mm, Phenomenex). Samples were pooled into 12 fractions.

Peptides were separated using an UltiMate 3000 RSLC nano LC system (Thermo Fisher Scientific) equipped with a trapping cartridge (Precolumn C18 PepMap 100, 5µm, 300 µm i.d. × 5 mm, 100 Å) and an analytical column (Acclaim PepMap 100, 75 µm × 50 cm C18, 3 pm, 100 Å). The LC system was directly coupled to a Q Exactive Plus mass spectrometer (ThermoFisher Scientific) using a Nanospray-Flex ion source. Using a 120 min gradient of solvent B (99.9% ACN, 0.1% formic acid) peptides were eluted and subjected to tandem mass spectrometry. The mass spectrometer was operated in Top 10 mode and dynamic exclusion was applied for 30 seconds.

For STPP-UP, pooled samples were acidified and desalted using StageTips (ThermoFisher Scientific) and eluted with 2× 30 µl of buffer B (80% acetonitrile, 0.01% trifluoroacetic acid (TFA)). Samples were fractionated using the Pierce^{™} High pH Reversed-Phase Peptide Fractionation Kit (ThermoFisher Scientific) into 4 fractions and cleaned up using StageTips. Peptides were eluted and applied to reverse-phase chromatography using a nanoLC-Easy1000 coupled online to a Thermo Orbitrap Q-Exactive HF-X. Using a 120 min gradient of buffer B, peptides were eluted and subjected to tandem mass spectrometry. The mass spectrometer was operated in Top20 mode and dynamic exclusion was applied for 30 seconds.

### STPP- UP data analysis

Raw MS files were analyzed using MaxQuant (version 2.1.4.0). Data were searched against either human or mouse UniProt database (downloaded 27-06-2017), with default settings and either TMT10plex or TMTpro 16plex enabled. MaxQuant output files were further processed in R (version 4.1.3). Data were filtered for reverse hits, potential contaminants, proteins only identified by site and protein quantified with less than two unique peptides. Proteins that did not have a signal in all channels were also filtered out. Data were background corrected and normalized by variance stabilizing transformation (vsn). Proteins with an Benjamini-Hochberg corrected p-value <0.05 and a FC >log2(0.5) after differential enrichment analysis using the DEP (Zhang et al., 2018. Nat Protoc 13: 530-550) package (version 1.16.0) were deemed significant. Carrier correction was done by adding the log2 fold changes between compound and control from the carrier batch to the log2 transformed signal intensities of the control samples from the test batch.

### TPP data analysis

Raw MS files were analyzed using IsobarQuant. Identification of peptide and protein was performed with Mascot against the mouse Uniprot database, modified to include known contaminants and the reversed protein sequences. Search parameters: trypsin, missed cleavages 3, peptide tolerance 10ppm, 0.02Da for MS/MS tolerance. Fixed modifications were carbamidomethyl on cysteines and TMT10plex on lysine; variable modifications included acetylation on protein N-terminus, oxidation of methionine, and TMT10plex on peptide N-termini). Output files were loaded into R, merged, filtered for duplicates and proteins with less than 2 unique peptides and saved in an ExpressionSet R-object. Potential batch effects were removed using limma (Ritchie et al., 2015. Nucleic Acids Res 43: e47) and data were normalized using vsn (Huber et al., 2002. Bioinformatics 18: S96-104). Normalization was done for each temperature independently, to account for the decreasing signal intensity at the higher temperatures. The abundance score of each protein was calculated as the average log2 fold change at the two lowest temperatures (37°C, 40.4°C). The stability score of each protein was calculated by subtracting the abundance score from the log2 fold changes of all temperatures and calculating the sum of the resulting values. The significance of abundance and thermal stability scores was assessed using a limma analysis, followed by an FDR analysis using the fdrtool package.

To test our method, we performed STPP-UP on E14 mouse embryonic stem cells (mESC) treated with the PRC2 inhibitor EED226 (Qi et al., 2017. Nat Chem Biol 13: 381-388) and a Tmax set at 57 °C, and compared the results to a conventional TPP experiment (Figure 2A, 2B, Figure 4A). Samples in the TPP experiment were TMT-labeled per temperature (2D-TPP2) to allow for the most accurate comparison between conditions at each temperature point. Stability scores were calculated as the sum of the log-transformed fold changes measured at each temperature point adjusted to the relative fold changes measured at the first two temperatures, which represent differences in base protein abundance level. We observe reduced denaturation of the FED protein at higher temperatures in the EED226-treated cells (Figure 4A) and an increased stabilization of the protein in both the TPP and STPP-UP experiments (Figure 2A, 2B), confirming the direct binding of EED226 to the EED protein. We also observe slight stabilization of UNC119B in both STPP-UP and TPP experiments, which is likely an off-target effect as UNC119B has no known polycomb-related functions. We identify several proteins that show stabilization in our STPP-UP experiment, which do not show an altered stability in our TPP experiment. It should be noted that these proteins only show stabilization after carrier-correction (Figure 4B) and are therefore less reliable.

We also validated STPP-UP in human cells, using the anticancer drug C1 (van der Krift et al., 2023. BioRxiv 10.1101/2023.02.26.530079) on the LS 174T colorectal cancer cell line (Figure 2C, 2D, Figure 4C). In line with previously published TPP data (van der Krift et al., 2023. BioRxiv 10.1101/2023.02.26.530079), we successfully identify DHFR as the primary target for C1 using STPP-UP. While the target-to-background enrichment is not as strong as in the mouse STPP-UP experiment, and there appear to be more off-targets, these effects can mostly be attributed to carrier-correction, as enrichment of DHFR becomes much more pronounced upon removal of these correction effects (Figure 4D).

For both our mouse and human STPP-UP experiments, we chose a Tmax of 57 °C as the end point of the heat treatment. Analysis of previously published TPP data (Kumar et al., 2022. Cell Oncol 10.1007/s13402-022-00753-x; van der Krift et al., 2023. BioRxiv 10.1101/2023.02.26.530079)) and our own TPP experiment as performed in Figure 2A shows an average Tm-50 (melting temperature at which 50% of the protein is degraded relative to the lowest temperature) of 51 °C for mouse and 52 °C for human (Figure 5). As protein denaturation increases with temperature (Figure 6A) and because the time spent at each temperature in the STPP-UP temperature increment is relatively short, we reasoned that using a Tmax higher than Tm-50 would leave us with a total amount of protein equal to the amount of protein at Tm-50. We tested how the protein abundance for different Tmax used in STPP-UP would compare to TPP (Figure 6B) and found that the STPP-UP equivalent of a TPP Tm-50 of 52 °C would be around 57 °C. To confirm, we performed STPP-UP using EED226 in R1 mESCs with different Tmax, and did not see enrichment of EED in any Tmax other than 57 °C (Figure 7).

To summarize, this work reports an improved method for detecting changes in protein stability, reducing both the amount of input material and instrument time required 10-fold compared to conventional TPP. We are able to use this improved method to accurately determine the direct targets of drug compounds in both mouse and human. An argument for choosing STPP-UP over performing TPP at a single temperature, e.g. Tm-50, is that STPP-UP evaluates differences in protein denaturation rate at multiple temperatures and compounds the differences in protein abundance found at each intermediate temperature, all in one sample. In the future, we envision broad application for STPP-UP in the fields of drug development and cancer research.

**Table 1. Overview of labels used. All samples in the same box were pooled and measured in a single experiment**

| **Experiment identifier** | **Test/Carrier** | **Treatment** | **Replicate** | **TMT lable** | **Tmax** |
|---|---|---|---|---|---|
| DZ064 | Test | DMSO | 1 | 126 | 57 |
| DZ064 | Test | DMSO | 2 | 127N | 57 |
| DZ064 | Test | DMSO | 3 | 127C | 57 |
| | | EED226 | | | |
| DZ064 | Test | 10µM EED226 | 1 | 130N | 57 |
| DZ064 | Test | 10µM EED226 | 2 | 130C | 57 |
| DZ064 | Test | 10µM | 3 | 131 | 57 |
| DZ064 | Carrier | DMSO | 1 | 126 | 57 |
| DZ064 | Carrier | DMSO | 2 | 127N | 57 |
| DZ064 | Carrier | DMSO EED226 | 3 | 127C | 57 |
| DZ064 | Carrier | 10µM EED226 | 1 | 130N | 57 |
| DZ064 | Carrier | 10µM EED226 | 2 | 130C | 57 |
| DZ064 | Carrier | 10µM | 3 | 131 | 57 |
| DZ078 | Test | DMSO | 1 | 126 | 57 |
| DZ078 | Test | DMSO | 2 | 127N | 57 |
| DZ078 | Test | DMSO | 3 | 127C | 57 |
| DZ078 | Test | C1 10µM | 1 | 128N | 57 |
| DZ078 | Test | C1 10µM | 2 | 128C | 57 |
| DZ078 | Test | C1 10µM | 3 | 129N | 57 |
| DZ078 | Carrier | DMSO | 1 | 129C | 57 |
| DZ078 | Carrier | DMSO | 2 | 130N | 57 |
| DZ078 | Carrier | DMSO | 3 | 130C | 57 |
| DZ078 | Carrier | C1 10µM | 1 | 131N | 57 |
| DZ078 | Carrier | C1 10µM | 2 | 131C | 57 |
| DZ078 | Carrier | C1 10µM | 3 | 132N | 57 |
| DZ071 - A | Test | DMSO | 1 | 126 | 52 |
| DZ071 - A | Test | DMSO | 2 | 127N | 52 |
| DZ071 - A | Test | DMSO | 3 | 127C | 52 |
| DZ071 - A | Test | C1 10µM | 1 | 128N | 52 |
| DZ071 - A | Test | C1 10µM | 2 | 128C | 52 |
| DZ071 - A | Test | C1 10µM | 3 | 129N | 52 |
| DZ071 - A | Carrier | DMSO | 1 | 129C | 52 |
| DZ071 - A | Carrier | DMSO | 2 | 130N | 52 |
| DZ071 - A | Carrier | DMSO | 3 | 130C | 52 |
| DZ071 - A | Carrier | C1 10µM | 1 | 131N | 52 |
| DZ071 - A | Carrier | C1 10µM | 2 | 131C | 52 |
| DZ071 - A | Carrier | C1 10µM | 3 | 132N | 52 |
| DZ071 - B | Test | DMSO | 1 | 126 | 57 |
| DZ071 - B | Test | DMSO | 2 | 127N | 57 |
| DZ071 - B | Test | DMSO | 3 | 127C | 57 |
| DZ071 - B | Test | C1 10µM | 1 | 128N | 57 |
| DZ071 - B | Test | C1 10µM | 2 | 128C | 57 |
| DZ071 - B | Test | C1 10µM | 3 | 129N | 57 |
| DZ071 - B | Carrier | DMSO | 1 | 129C | 57 |
| DZ071 - B | Carrier | DMSO | 2 | 130N | 57 |
| DZ071 - B | Carrier | DMSO | 3 | 130C | 57 |
| DZ071 - B | Carrier | C1 10µM | 1 | 131N | 57 |
| DZ071 - B | Carrier | C1 10µM | 2 | 131C | 57 |
| DZ071 - B | Carrier | C1 10µM | 3 | 132N | 57 |
| DZ071 - C | Test | DMSO | 1 | 126 | 62 |
| DZ071 - C | Test | DMSO | 2 | 127N | 62 |
| DZ071 - C | Test | DMSO | 3 | 127C | 62 |
| DZ071 - C | Test | C1 10µM | 1 | 128N | 62 |
| DZ071 - C | Test | C1 10µM | 2 | 128C | 62 |
| DZ071 - C | Test | C1 10µM | 3 | 129N | 62 |
| DZ071 - C | Carrier | DMSO | 1 | 129C | 62 |
| DZ071 - C | Carrier | DMSO | 2 | 130N | 62 |
| DZ071 - C | Carrier | DMSO | 3 | 130C | 62 |
| DZ071 - C | Carrier | C1 10µM | 1 | 131N | 62 |
| DZ071 - C | Carrier | C1 10µM | 2 | 131C | 62 |
| DZ071 - C | Carrier | C1 10µM | 3 | 132N | 62 |
| DZ071 - D | Test | DMSO | 1 | 126 | 67 |
| DZ071 - D | Test | DMSO | 2 | 127N | 67 |
| DZ071 - D | Test | DMSO | 3 | 127C | 67 |
| DZ071 - D | Test | C1 10µM | 1 | 128N | 67 |
| DZ071 - D | Test | C1 10µM | 2 | 128C | 67 |
| DZ071 - D | Test | C1 10µM | 3 | 129N | 67 |
| DZ071 - D | Carrier | DMSO | 1 | 129C | 67 |
| DZ071 - D | Carrier | DMSO | 2 | 130N | 67 |
| DZ071 - D | Carrier | DMSO | 3 | 130C | 67 |
| DZ071 - D | Carrier | C1 10µM | 1 | 131N | 67 |
| DZ071 - D | Carrier | C1 10µM | 2 | 131C | 67 |
| DZ071 - D | Carrier | C1 10µM | 3 | 132N | 67 |

## Claims

1. A method of identifying at least one protein that exhibits altered thermal stability upon addition a compound, comprising the steps of:
a) incubating at least one container comprising a plurality of proteins with the compound, and at least one container comprising the plurality of proteins with a control;
b) exposing the containers to an increasing temperature;
c) digesting the proteins;
d) labelling the digested proteins; and
e) comparing the labelled digested proteins from the at least one container that was incubated with the compound with the labelled digested proteins from the at least one container that was incubated with the control, to identify at least one protein that exhibits altered thermal stability upon addition of the compound.

2. The method of claim 1, wherein step b) is followed by a step of separating soluble from insoluble proteins, followed by digesting the soluble and/or the insoluble proteins in step c).

3. The method of claim 1 or 2, wherein an aliquot, termed carrier, is removed from the at least one container that was incubated with the compound after step a), which aliquot is used to adjust for differences between replicates, while an equal aliquot is removed from the at least one container that was incubated with the compound after step a) and is termed tester.

4. The method of claim 3, wherein the carrier aliquot is incubated at a temperature between 20 and 50 °C for between 10 seconds and 10 minutes, such as between 30 seconds and 5 minutes, including 1 minute, 2 minutes, 3 minutes, and 4 minutes.

5. The method of any one of claims 1-4, comprising identifying at least one protein that exhibits altered thermal stability upon addition of a compound for a plurality of conditions whereby, for each condition, at least one container comprising a plurality of proteins is incubated with the compound, and at least one container comprising the plurality of proteins is incubated with a control.

6. The method of claim 5, whereby, for each condition, an aliquot is removed from the at least one container that was incubated with the compound after step a), which aliquot is used to adjust for differences between conditions and, if appropriate, between replicates.

7. The method of any one of claims 1-6, wherein the plurality of proteins is present in one or more cells such as mammalian cells, including human cells.

8. The method of any one of claims 1-7, wherein the at least one protein interacts with the compound.

9. The method of any one of claims 1-8, wherein the control is or comprises the solvent in which the compound is solubilized, such as dimethyl sulfoxide.

10. The method of any one of claims 1 to 9, wherein the digested proteins are labelled with a tandem mass tag (TMT) label.

11. The method of any one of claims 1 to 10, wherein all labelled samples are combined before comparing the labelled digested proteins from the at least one container that was incubated with the compound with the labelled digested proteins from the at least one container that was incubated with the control, to identify at least one protein that exhibits altered thermal stability upon addition of the compound.

12. The method of any one of claims 1 to 11, wherein the comparison of the labelled digested proteins from the at least one container that was incubated with the compound with the labelled digested proteins from the at least one container that was incubated with the control, is performed by mass spectrometry, such as reverse-phase liquid chromatography (RP-LC) coupled to tandem mass spectrometry (MS/MS).

13. The method of any one of claims 1 to 12, wherein the tester aliquots are exposed to an increasing temperature between 30 °C and 75 °C, such as between 36 °C and 58 °C, between 37 °C and 57 °C, between 38 °C and 56 °C, between 39 °C and 55 °C, or between 40 °C and 54 °C.

14. The method of any one of claims 1 to 13, wherein the tester aliquots are exposed to a steadily increasing temperature between 30 °C and 75 °C, at a rate between 0.05 °C and 5 °C per second.

15. The method of any one of claims 1 to 14, wherein the proteins are digested with a combination of trypsin and LysC endoproteinase.
